# EUROPÄISCHE PATENTANMELDUNG

(11) **EP 1 024 362 A2**
(43) Veröffentlichungstag der Anmeldung: **02.08.2000**
(21) Anmeldenummer: 00101469.5
(22) Anmeldetag: 26.01.2000
(51) Int. Cl.: G01N 33/487, B01L 3/00, G01N 27/416, A61M 1/14, A61B 5/00

(54) **Aufnahmeeinheit für Lösungen, insbesondere Lösungen zur Kalibrierung von Sensoren zur Messung physiologisch relevanter Parameter**

(30) Priorität: 30.01.1999 DE 19903704
(71) Anmelder: Fresenius Medical Care Deutschland GmbH, 61352 Bad Homburg v.d.H. (DE)
(72) Erfinder: König, Christoph, 65207 Wiesbaden-Auringen (DE); Mager, Gerhard, Dr., 61352 Bad Homburg v.d.H. (DE); Abel, Petra, 61169 Friedberg (DE)
(74) Vertreter: Luderschmidt, Schüler & Partner GbR

(57) **Zusammenfassung**

Eine zur einmaligen Verwendung bestimmte Aufnahmeeinheit für Lösungen, insbesondere Lösungen zur Kalibrierung von Sensoren zur Messung physiologisch relevanter Parameter, die als Blisterverpackung ausgebildet ist. Sie umfaßt mehrere Kammern (4) zur Aufnahme der Lösungen, wobei jede Kammer über einen Auslaßkanal (6), der von einem Verschlußorgan (13) verschlossen ist, mit einem Probenkanal (7) verbunden ist. Die Sensoren (18) zur Messung der physiologischen Parameter sind vorzugsweise Bestandteil der Aufnahmeeinheit. Die zu analysierende Probe wird maschinenseitig abgezogen und dem Probenkanal zugeführt. Zur Kalibrierung der Sensoren werden die in den Kammern befindlichen Kalibrierlösungen nach Öffnen der entsprechenden Verschlußorgane in den Probenkanal dosiert. Nach der Behandlung wird die Aufnahmeeinheit zusammen mit den Sensoren entsorgt.

## Beschreibung

Die Erfindung betrifft eine Aufnahmeeinheit für Lösungen, insbesondere Lösungen zur Kalibrierung von Sensoren zur Messung physiologisch relevanter Parameter, die als austauschbares Modul ausgebildet und zur einmaligen Verwendung bestimmt ist.

In der Medizintechnik finden Sensoren zur Messung physiologisch relevanter Paramenter, beispielsweise Harnstoff während einer Dialysebehandlung Verwendung. Da ihre Betriebsparamater sowohl durch Herstellung als auch Lagerung undefinierten Streuungen unterliegen, ist eine Kalibrierung dieser Sensoren erforderlich.

Zur Kalibrierung müssen den Sensoren zur Messung physiologisch relevanter Pararmater kleine Volumina von Kalibrierlösungen zugeführt werden. Die Applikation der Kalibrierlösungen kann manuell oder mechanisch erfolgen.

Die DE 33 12 923 A1 beschreibt eine Aufnahmeeinheit zur elektrochemischen Analyse elektrolytischer Bestandteile einer flüssigkeit, insbesondere Blut oder Urin, die über eine Probenkammer zur Aufnahme der Flüssigkeit und eine Elektrodenanordnung verfügt. Die Probenkammer ist herstellerseitig mit einer Standard-Elektrolytlösung befällt, die zur Vorkonditionierung der Elektrodenanordnung dient. Nach der Entleerung der Probenkammer wird diese mit der zu analysierenden flüssigkeit befüllt. Zur Durchführung der Analyse wird die Aufnahmeeinheit dann in ein Analysegerät eingesetzt.

Aus der EP 0 354 895 B1 ist ein aus einem Sensorteil und einem Probennahmeteil bestehendes Einweg-Meßelement bekannt, das in ein Analysegerät eingesetzt wird. Der Sensorteil weist einen mit Senoren bestückten Meßkanal auf, der herstellerseitig mit einer Kalibrierlösung befüllt ist. Wenn das Einweg-Meßelement in das Analysegerät eingesetzt ist, wird die Kalibrierlösung aus dem Meßkanal des Sensorteils abgezogen und die zu analysierende Flüssigkeit aus dem Probenahmeteil in den Meßkanal angesaugt.

Eine als Disposable ausgebildete Aufnahmeeinheit zur Analyse von Blutproben, die einen herstellerseitig mit Kalibrierlösung befüllten Meßkanal aufweist, ist auch aus der WO 86/05590 bekannt.

Die WO 85/04719 offenbart eine Einschubkarte für ein Analysegerät, die über einen mit Sensoren bestückten Probenkanal verfügt. Auf der Karte befindet sich ein Sockel, in dem ein Zylinder mit zwei Kammern drehbar gelagen ist. Die eine Kammer ist herstellerseitig mit einer Kalibrierlösung befüllt, während die andere Kammer die zu analysierende Flüssigkeit aufnimmt. Der Zylinder kann zwischen zwei Positionen gedreht werden, wobei in der einen Position die mit Kalibrierlösung befüllte Kammer und in der anderen Position die Kammer zur Aufnahme der zu analysierenden Flüssigkeit mit dem Probenkanal verbunden ist.

Aus der US-A-5,096,669 ist eine Einschubkarte für ein Analysegerät bekannt, bei der die Kalibrierlösung für den Sensor in einem kleinen Folienbeutel verpackt zur Verfügung gestellt wird. Der in einer muldenförmigen Vertiefung liegende Beutel wird mittels eines Dorns durchstoßen, wobei die Kalibrierlösung dann durch in der Einschubkarte ausgebildete Kanäle zu den Sensoren gelangt.

Zur Messung der während einer Dialysebehandlung relevanten physiologischen Parameter sind mehrere Sensoren erforderlich. Zur Kalibrierung dieser Sensoren müssen also mehrere Kalibrierlösungen zur Verfügung gestellt werden.

Ein Applikationssystem mit mehreren Kammern zur Aufnahme von Medikamenten ist aus der DE 197 02 362 A1 bekannt. Das Applikationssystem weist einen schalenförmigen Behälter mit einem Zulauf- und Ablaufstutzen für eine Infusionslösung auf. In dem Behälter sitzt ein Dosiereinsatz, der über zylindrische Kammern verfügt, die mit einer Verschlußfolie dicht verschlossen sind. Nach dem Durchbrechen der Verschlußfolie können die Kammern in einen Spaltraum entleert werden, der von der Infusionslösung durchströmt wird. Als nachteilig erweist sich bei dem bekannten Applikationssystem die relativ aufwendige Herstellung.

Die US-A-4,871,439 beschreibt ein Disposable mit einem Probenkanal, an dem mehrere Sensoren zur Bestimmung charakteristischer Eigenschaften einer Körperflüssigkeit angeordnet sind. Das Disposable umfaßt zwei Kammern zur Aufnahme von Kalibrierlösungen für die Sensoren. Die Kammern, die die Kalibrierlösungen enthalten, bestehen aus an ihrem Kanten heiß versiegelten Aluminiumfolien. Die Enbindung dieser Kammern in das Disposable ist relativ aufwendig.

Eine Einheit zur Untersuchung von biologischen Flüssigkeiten, bei der als Vorratsbeutel für die Systemlösung sowie für den Abfall verschweißte PE-Folien verwendet werden, ist aus der DE-295 11 566 U1 bekannt. Die US-A-5,284,570 offenbart eine nach Art einer Spritze ausgebildete Analyseneinheit. Eine Analyseneinheit mit einem Kalibrierlösungsbehälter, der von einer durchstechbaren Membran verschlossen ist, beschreibt die US-A- 5,421,981.

Der Erfindung liegt die Aufgabe zugrunde, eine zur einmaligen Verwendung bestimmte und als austauschbares Modul ausgebildete Aufnahmeeinheit zu schaffen, mit der mehrere Lösungen, insbesondere Lösungen zur Kalibrierung von Sensoren zur Messung physiologisch relevanter Parameter auf einfache Weise unter sterilen Bedingungen zur Verfügung gestellt werden können, wobei die Aufnahmeeinheit sich in großen Stückzahlen mit verhältnismäßig geringem Aufwand herstellen läßt.

Die Lösung dieser Aufgabe erfolgt erfindungsgemäß mit den im Patentanspruch 1 angegebenen Merkmalen.

Die nach Art einer Blisterverpackung ausgebildete Aufnahmeeinheit kann in großen Stückzahlen als Einmalartikel kostengünstig hergestellt werden. Die Aufnahmeeinheit weist einen Basiskörper auf, wobei die Kammern von einer Hülle aus flexiblem Material begrenzt werden, die zusammen mit dem Basiskörper abgeschlossene Hohlräume bildet. Da die Kammern zur Aufnahme der Lösungen ein definiertes Volumen haben, ist eine zusätzliche Meß- bzw. Dosiereinrichtung nicht erforderlich.

Die nach Art einer Blisterverpackung ausgebildete Aufnahmeeinheit hat eine flache Bauweise und läßt sich als austauschbares Modul einfach handhaben. Der Basiskörper der Aufnahmeeinheit kann eine Vielzahl von Kammern auf engstem Raum aufnehmen. Eine Kontamination der Lösungen in der Aufnahmeeinheit ist ausgeschlossen. Auch können keine Verunreinigungen aus der Umgebung in die Aufnahmeeinheit eingetragen werden, wenn einzelnen Kammern entleert sind.

Um eine hohe Packungsdichte zu erzielen, sind die Kammern vorzugsweise in einer Reihe nebeneinanderliegend angeordnet. Es können auch mehrere Reihen von Kammern vorgesehen sein. In diesem Fall ist für jede Reihe ein Verbindungskanal vorgesehen, der den Auslaßkanal jeder Kammer einer Reihe mit dem Probenkanal verbindet.

Die erfindungsgemäße Aufnahmeeinheit dient insbesondere zur portionsweisen Verpackung von mehreren, d.h. mindestens zwei Lösungen zur Kalibrierung von Sensoren zur Messung physiologisch relevanter Parameter. Sie kann aber beispielsweise auch zur Aufnahme von Kontroll-, Konditionier-, Reinigungs- und Desinfektionsiösungen verwendet werden.

Die erfindungsgemäße Aufnahmeeinheit erlaubt eine zuverlässige Handhabung kleiner Flüssigkeitsmengen. Die Lösungen werden in Einzelportionen verpackt zur Verfügung gestellt und können der Reihe nach in einen Probenkanal dosiert werden. Falls erforderlich kann die Reihenfolge, in der die Lösungen in den Probenkanal dosiert werden, durch geeignete Anordnung der Kammern in der Aufnahmeeinheit vorgegeben werden.

In einer bevorzugten Ausführungsform der Aufnahmeeinheit, die zur Kalibrierung von Sensoren insbesondere zur Messung physiologisch relevanter Parameter verwendet wird, sind die zu kalibrierenden Sensoren Bestandteil der Aufnahmeeinheit. Die Aufnahmeeinheit verfügt über einen Anschluß zum Zuführen einer mit den Sensoren zu analysierenden Probe in den Probenkanal und einen Anschluß zum Abführen der Probe aus dem Probenkanal. Zur Kalibrierung der Sensoren werden die Kalibrierlösungen aus den Kammern in den Probenkanal dosiert.

Die Anordnung der Sensoren auf der Aufnahmeeinheit hat den Vorteil, daß eine Reinigung, Desinfektion oder Sterilisation derselben nicht erforderlich ist. Nach dem einmaligen Gebrauch werden die Sensoren zusammen mit der Aufnahmeeinheit verworfen. Dies ist insofern besonders vorteilhaft, als elektrochemische Sensoren auf nahezu alle Reinigungs- und Desinfektionsmittel mit erheblicher Beeinträchtigung oder sogar dem Verlust der Funktion reagieren. Im übrigen wäre die Reinigung, Desinfektion oder Sterilisation der Sensoren in der praktischen Anwendung sehr arbeitsaufwendig und damit unpraktikabel. Durch die zur einmaligen Verwendung bestimmte Aufnahmeeinheit, die auch die Sensoren umfaßt, wird dieses Problem mit einfachen Mitteln und großer Sicherheit gelöst.

Zur Herstellung einer elektrischen Verbindung zwischen den Sensoren und dem Analysegerät, in das sich das Modul einsetzen läßt, verfügt die Aufnahmeeinheit vorteilhafterweise über Kontakte, an denen die Sensoren angeschlossen sind.

Die Kammern zur Aufnahme der Lösungen können unterschiedlich ausgebildet sein. Besonders vorteilhaft ist aber, wenn die Kammern derart ausgebildet sind, daß eine Entleerung derselben mittels Betätigungsorganen möglich ist, die an den Kammern von außen angreifen. Derartige Betätigungsorgane können dann Bestandteil des Analysegerätes sein.

In bevorzugter Ausgestaltung der Erfindung ist ein an der Rückseite der Basisplatte unterhalb der Kammern verlaufender Verbindungskanal vorgesehen, über den die Auslaßöffnungen der Kammern mit dem Probenkanal verbunden sind. Die Auslaßöffnungen sind hier Bohrungen in der Basisplatte.

Der Verbindungs- und Probenkanal werden zweckmäßigerweise durch Rinnen auf der Rückseite der Basisplatte gebildet, die von einer auf die Rückseite der Basisplatte aufgebrachten Folie abgedeckt sind.

In dem Basiskörper können unterhalb der Kammern noch weitere Bohrungen vorgesehen sein, die von der Abdeckfolie auf der Rückseite des Basiskörpers verschlossen sind. Diese Bohrungen dienen dazu, die Kammern vor dem Aufbringen der Folie auf die Rückseite des Basiskörpers mit den Lösungen zu befüllen.

Die Herstellung der Aufnahmeeinheit kann noch damit weiter vereinfacht werden, daß die Auslaßöffnung der Kammer mit einem Teil verschlossen wird, das über eine Sollbruchstelle an den Basiskörper angeschlossen ist. Der Basiskörper kann somit zusammen mit den Verschlußorganen als Spritzgießteil hergestellt werden.

Das den Auslaßkanal jeder Kammer verschließende Verschlußorgan kann aber auch eine Folie sein, die auf die Oberseite des Basiskörpers aufgebracht ist und den Auslaßkanal verschließt. Prinzipiell kann auch eine auf die Rückseite des Basiskörpers aufgebrachte Folie zum Verschließen der Verbindungsbohrung vorgesehen sein.

Zur Herstellung der Strömungsverbindung zwischen den Kammern und dem Probenkanal wird die Verschlußfolie vorteilhafterweise mit einem Stempel durchstoßen, der in jeder Kammer vorgesehen ist. Die Kammern sind vorzugsweise derart ausgebildet, daß die Stempel zum Durchdrücken der Verschlußfolie in den Kammern fixiert sind.

Im folgenden wird ein Ausführungsbeispiel der Erfindung unter Bezugnahme auf die Zeichnungen näher erläutert.

Es zeigen:
- Figur 1: eine erste Ausführungsform einer Aufnahmeeinheit für Kalibrierlösungen in der Draufsicht,
- Figur 2: einen Schnitt durch die Aufuahmeeinheit von Figur 1 entlang der Linie II-II,
- Figur 3: die Aufnahmeeinheit von Figur 1 in der Unteransicht und
- Figur 4: eine Teilansicht einer weiteren Ausführungsform der Auf nahmeeinheit in vergrößerter Darstellung.

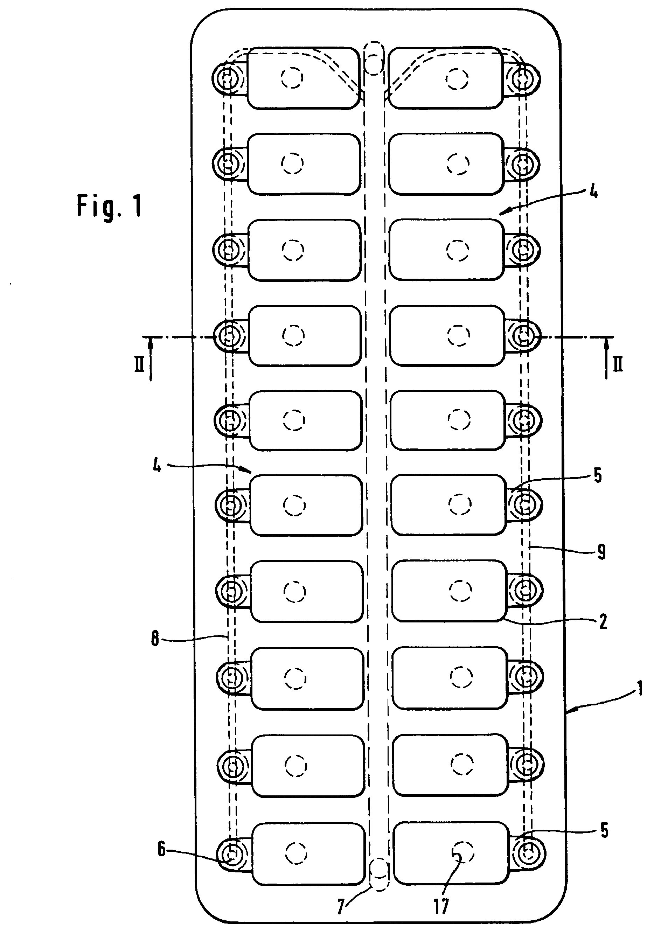

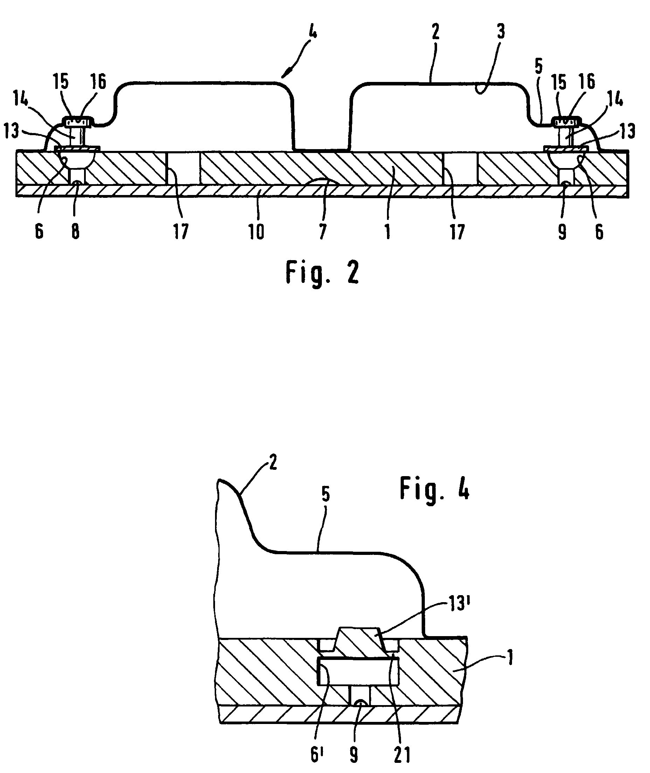

Die Figuren 1 bis 3 zeigen eine nach Art einer Blisterverpackung ausgebildete Aufnahnieeinheit für Lösungen zur Kalibrierung von elektrochemischen Sensoren zur Messung physiologisch relevanter Parameter, beispielsweise zur Harnstoffmessung während einer Dialysebehandlung. Die Aufnahmeeinheit umfaßt eine rechteckförmige Platte 1 aus transparentem Kunststoff. Auf die Oberseite der Kunststoffplatte ist eine transparente Kunststoffolie 2 aufgebracht, die unter Bildung von zwei nebeneinander angeordneten Reihen von jeweils zehn muldenförmigen Vertiefungen 3 ausgeformt ist. Die vorzugweise mit der Kunststoffplatte 1 verschweißte oder verklebte Kunststoffolie 2 begrenzt zusammen mit der Kunststoffplatte insgesamt zwanzig Kammern 4. Die Kammern 4 haben eine im wesentlichen rechteckförmige Grundfläche. An ihrer Schmalseite weisen die Kammern 4 ein nach außen weisenden Ansatz 5 auf, der eine geringere Höhe als der übrige Teil der Kammer hat.

Im Bereich der flachen Ansätze 5 der Kammern sind in der Kunststoffplatte 1 Verbindungsbohrungen 6 vorgesehen. An der Rückseite weist die Kunststoffplatte 1 eine in Längsrichtung zwischen den Kammern 4 verlaufende mittige Rinne 7 und zwei seitliche Rinnen 8, 9 auf, die unterhalb der flachen Ansätze 5 der Kammern 4 entlang der Linien verlaufen, auf denen die Verbindungsbohrungen 6 liegen. Die seitlichen Rinnen 8, 9 münden an einer Schmalseite der Kunststoffplatte in die mittige Rinne 7.

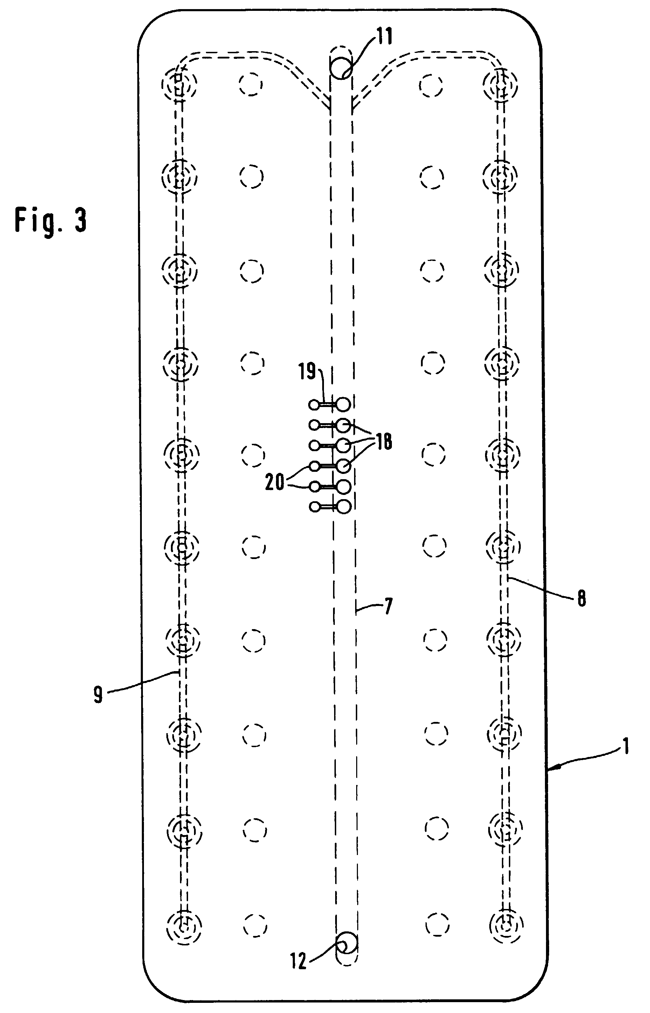

Mit der Rückseite der Kunststoffplatte 1 ist eine Abdeckfolie 10 verschweißt oder verklebt, so daß zwei seitliche Verbindungskanäle 8, 9 und ein mittiger Probenkanal 7 geschaffen werden. An dem einen Ende des Probenkanals 7 weist die Abdeckfolie 10 einen Anschluß 11 zum Zuführen einer zu analysierenden Probe und an dem anderen Ende des Probenkanals einen Anschluß 12 zum Abführen der Probe auf (Figur 3).

Die Verbindungsbohrungen 6 in der Kunststoffplatte 1 sind mit Verschlußelementen 13 verschlossen. Die Verschlußelemente sind kreisförmige Folienstücke, die in den flachen Ansätzen 5 der Kammern 4 auf die Oberseite der Kunststoffplatte 1 aufgebracht sind. Zum Öffnen der Verschlußelemente sind in die flachen Ansätze 5 der Kammern 4 Stempel 14 eingesetzt. Die Stempel 14 weisen einen runden Kopf 15 auf, der in einer entsprechenden Ausformung 16 der Folie sitzt. Sie werden zwischen der Folie 4 und der Kunststoffplatte 1

leicht klemmend gehalten. Die Kammern der Aufnahmeeinheit weisen ein Volumen von etwa 1 ml auf und sind mit unterschiedlichen Kalibrierlösungen befüllt. Um die Kammern befüllen zu können, sind in der Kunststoffplatte 1 im Bereich der Kammern weitere Bohrungen 17 vorgesehen, durch die sich die Reagenzien vor dem Aufbringen der Abdeckfolie 10 auf die Rückseite der Platte in die Kammern einfüllen lassen.

Die ionenselektiven Sensoren 18 zur Messung der physiologischen Parameter einer durch den Probenkanal 7 strömenden Flüssigkeit befinden sich auf der Rückseite der Kunststoffplatte 1. Die elektrischen Anschlußkontakte der Sensoren sind über Verbindungsleitungen 19 mit einer Reihe von Kontakten 20 verbunden, die sich an der Rückseite der Abdeckfolie 10 befinden.

Die als austauschbares Modul ausgebildete Aufnahmeeinheit wird in ein nicht dargestelltes Analysegerät mit einem die elektrischen Signale der Sensoren verarbeitenden Meßelektronik eingesetzt. Das Analysegerät verfügt sowohl über vorzugsweise hydraulische Konnektoren für die Anschlüsse 11, 12 der Aufnahmeeinheit zum Zuführen und Abführen der Probe als auch elektrische Kontakte zur Herstellung einer elektrischen Verbindung mit den entsprechenden Kontakten 20 der Aufnahmeeinheit.

Die zu messende Probe wird maschinenseitig abgezogen, durch den Probenkanal 7 über die Sensoren 18 geleitet und verworfen. Die Sensoren geben dann die Meßwerte an das Analysegerät der Dialysevorrichtung ab, wo sie verarbeitet und/oder dargestellt werden.

Zur Kalibrierung der Sensoren 18 können die in den einzelnen Kammern befindlichen Kalibrierlösungen der Reihe nach in den Probenkanal 7 dosiert werden. Hierzu wird der Stempel 14 in dem flachen Ansatz 5 der zu entleerenden Kammer 4 in die Verschlußfolie 13 gedrückt, wodurch diese durchstoßen wird. Die Lösung kann dann aus der Kammer über den seitlichen Verbindungskanal 8 bzw. 9 in den mittigen Probenkanal 7 fließen.

Zum Entleeren der Kammern verfügt das Analysegerät über eine Betätigungseinrichtung, die den einzelnen Kammern und Verschlußelementen zugeordnete Stößel aufweist. Die elektromagnetisch oder pneumatisch betätigbaren Stößel des Analysegeräts bestätigen die Verschlußorgane und drücken die zu entleerenden Kammern 4 zusammen, wodurch der Weg über die seitlichen Verbindungskanäle 8, 9 frei wird und die jeweilige Kalibrierlösung in den Probenkanal 7 gelangt. Bei jeder Kalibrierung steht also eine frische, sterile Kalibrierlösung zur Verfügung, die keine Kontamination erfahren hat. Am Ende der Behandlung wird die als Disposable ausgebildete Aufnahmeeinheit dann verworfen.

Figur 4 zeigt eine Teilansicht einer weiteren Ausführungsform der Aufnahmeeinheit. Dieses Ausführungsbeispiel unterscheidet sich von der unter Bezugnahme auf die Figuren 1 bis 3 beschriebenen Ausführungsform durch die Verschlußelemente 13', mit denen die Verbindungsbohrungen 6' in der Basisplatte 1 verschlossen sind. Die Verschlußelemente 13' sitzen in den Verbindungsbohrungen 6' und sind über schmale Stege 21 an den Basiskörper 1 angeschlossen. Wenn die Verschlußelemente 13' in die Verbindungsbohrungen 6' gedrückt werden, bricht der schmale Verbindungssteg, so daß die Kalibrierlösung aus der Kammer 4 in den Probenkanal 7 gelangt. Die Verschlußelemente 13' werden von den Stößeln der' Betätigungseinrichtung des Analysegeräts herausgebrochen, in das die Aufnahmeeinheit eingelegt wird.

## Patentansprüche

1. Aufnahmeeinheit für Lösungen, insbesondere Lösungen zur Kalibrierung von Sensoren zur Messung physiologisch relevanter Parameter, mit mehreren Kammern (4) zur Aufnahme der Lösungen, wobei jede Kammer eine Auslaßöffnung (6, 6') aufweist, die von einem Verschlußorgan (13, 13') verschlossen ist, und wobei ein Probenkanal (7) vorgesehen ist, der mit der Auslaßöffnung jeder Kammer verbunden ist, so daß nach dem Öffnen der Verschlußorgane die Lösungen in den Probenkanal überführbar sind, dadurch gekennzeichnet, daß die Aufnahmeeinheit einen plattenförmigen Basiskörper (1) aufweist, auf deren Oberseite eine Hülle (3) aus flexiblem Material unter Bildung der Kammern (4) aufgebracht ist.

2. Aufnahmeeinheit nach Anspruch 1, dadurch gekennzeichnet, daß die Hülle (3) aus flexiblem Material eine Kunststoffolie ist.

3. Aufnahmeeinheit nach Anspruch 1 oder 2, dadurch gekennzeichnet, daß auf der Rückseite der Basisplatte (1) ein unterhalb der Kammern (4) verlaufender Verbindungskanal (8,9) vorgesehen ist, der über jeweils eine Verbindungsbohrung (6) mit jeder Kammer verbunden ist, wobei der Probenkanal (7) an den Verbindungskanal (8,9) angeschlossen ist.

4. Aufnahmeeinheit nach Anspruch 3, dadurch gekennzeichnet, daß auf der Rückseite der Basisplatte (1) Rinnen ausgebildet sind, die von einer Folie (10) unter Bildung des Verbindungskanals (8,9) und des Probenkanals (17) abgedeckt sind, die auf die Rückseite der Basisplatte aufgebracht ist.

5. Aufnahmeeinheit nach Anspruch 4, dadurch gekennzeichnet, daß in der Basisplatte (1) unterhalb der Kammern (4) Bohrungen (17) zum Befüllen derselben vorgesehen sind, die von der Folie (10) an der Rückseite der Basisplatte verschlossen sind.

6. Aufnahmeeinheit nach einem der Ansprüche 1 bis 5, dadurch gekennzeichnet, daß das Verschlußorgan ein die Auslaßöffnung der Kammer (4) verschließendes Element (13') ist, das über eine Solibruchzone an den Basiskürper (1) angeschlossen ist.

7. Aufnahmeeinheit nach einem der Ansprüche 1 bis 5, dadurch gekennzeichnet, daß das Verschlußorgan eine die Auslaßöffnung (6) der Kammer (4) verschließende Folie (13) ist.

8. Aufnahmeeinheit nach Anspruch 7, dadurch gekennzeichnet, daß in jeder Kammer (4) ein Stempel (14) zum Durchdrücken der Verschlußfolie (13) vorgesehen ist.

9. Aufnahmeeinheit nach Anspruch 8, dadurch gekennzeichnet, daß jede Kammer (4) derart ausgebildet ist, daß der Stempel (14) in der Kammer fixiert ist.

10. Aufnahmeeinheit nach einem der Ansprüche 1 bis 9, dadurch gekennzeichnet, daß die Kammern (4) in einer Reihe angeordnet sind.

11. Aufnahmeeinheit nach einem der Ansprüche 1 bis 10, dadurch gekennzeichnet, daß die Kammern (4) in mehreren Reihen angeordnet sind, wobei für jede Reihe ein zu dem Probenkanal (7) führender Verbindungskanal (8,9) vorgesehen ist, der an dem Auslaßkanal (6) jeder Kammer einer Reihe angeschlossen ist.

12. Aufnahmeeinheit nach einem der Ansprüche 1 bis 11, dadurch gekennzeichnet, daß die Kammern (4) mit Lösungen zur Kalibrierung eines oder mehrerer Sensoren, insbesondere Sensoren zur Messung physiologisch relevanter Parameter, befüllt sind, wobei ein Anschluß (11) zum Zuführen einer mit den Sensoren zu analysierenden Probe in den Probenkanal (7) und ein Anschluß (12) zum Abführen der Probe aus dem Probenkanal vorgesehen sind.

13. Aufnahmeeinheit nach einem der Ansprüche 1 bis 12, dadurch gekennzeichnet, daß der oder die mit den Lösungen zur kalibrierenden Sensoren (18) Bestandteil der Aufnahmeeinheit sind.

14. Aufnahmeeinheit nach Anspruch 13, dadurch gekennzeichnet, daß Kontakte (20) vorgesehen sind, an der die Sensoren (18) zur Herstellung einer elektrischen Verbindung angeschlossen sind.
